(19) Europäisches Patentamt
European Patent Office
Office européen des brevets

(11) Publication number: **0 478 073 A2**

# (12) EUROPEAN PATENT APPLICATION

(21) Application number: **91202421.3**

(22) Date of filing: **19.09.91**

(51) Int. Cl.[5]: **C07B 41/08**, C07C 69/96, C07C 68/06

(30) Priority: **20.09.90 US 585457**

(43) Date of publication of application:
**01.04.92 Bulletin 92/14**

(84) Designated Contracting States:
**AT BE CH DE DK ES FR GB GR IT LI NL SE**

(71) Applicant: **UNION CARBIDE CHEMICALS AND PLASTICS COMPANY, INC.**
**39 Old Ridgebury Road**
**Danbury Connecticut 06817-0001(US)**

(72) Inventor: **King, Stephen Wayne**
**120 Teays Meadows**
**Scott Depot, West Virginia 25560(US)**
Inventor: **Ream, Bernard Claude**
**1807 Rolling Hills Road**
**Charleston, West Virginia 25314(US)**

(74) Representative: **Smulders, Theodorus A.H.J., Ir. et al**
**Vereenigde Octrooibureaux, Nieuwe Parklaan 97**
**NL-2587 BN Den Haag(NL)**

## (54) Processes for the preparation of carboxylated compounds.

(57) A process for preparing carboxylated compounds which comprises contacting an active hydrogen-containing compound with a $CO_2$ synthon in the presence of a mixed metal oxide catalyst or a modified bimetallic or polymetallic catalyst under conditions effective to produce the carboxylated compound.

EP 0 478 073 A2

## Related Applications

The following is a related, commonly assigned application, filed on an even date herewith: U.S. Patent Application Serial No. (D-16536); which is incorporated herein by reference.

## Brief Summary of the Invention

### Technical Field

This invention relates to a process for preparing carboxylated compounds which comprises contacting an active hydrogen-containing compound with a $CO_2$ synthon in the presence of a mixed metal oxide catalyst or a modified bimetallic or polymetallic catalyst under conditions effective to produce the carboxylated compound.

## Background of the Invention

Various processes for the production of carboxylated compounds are known. Fujinami, T. et al., Chemistry Letters, pp. 749-752, 1981, discloses the preparation of dialkyl carbonates by the heterogeneous reaction of solid potassium carbonate with alkyl bromides in dimethylformamide or dimethylsulfoxide in the presence of an organostannyl compound such as hexabutyldistannoxane or chlorotributylstannane.

U.S. Patent No. 3,980,690 describes a process in which an alcohol is carboxylated into the corresponding carbonate by reacting the alcohol with oxygen and carbon dioxide in the presence of a catalyst system in the heterogeneous phase by introducing the reactants into a reactor charged with a catalyst, such as the complex system formed by copper and 4-vinylpyridine, causing the reactants to flow over the catalyst, and then withdrawing the products of the reaction and unconsumed reactants from the reactor.

U.S. Patent No. 3,803,201 relates to the synthesis of dimethyl carbonate by the reaction of methanol with cyclic ethylene or 1,2-propylene carbonate wherein the desired product is removed as produced by distillation of the methanol-dimethyl carbonate azeotrope and is then separated from the azeotrope by low temperature crystallization and distillation.

U.S. Patent No. 4,307,032 discloses a process for preparing a dialkyl carbonate by contacting a glycol carbonate with an alcohol at an elevated temperature in the presence of a thallium compound.

U.S. Patent No. 4,671,041 discloses a process for the prepartion of ethylene glycol and dimethyl carbonate by reacting methanol and ethylene carbonate in the presence of a series of heterogeneous catalyst systems including ion exchange resins with tertiary amine, quaternary ammonium, sulfonic acid and carboxylic acid functional groups, alkali and alkaline earth silicates impregnated into silica and ammonium exchanged zeolites.

U.S. Patent No. 4,734,518 describes a process for the cosynthesis of ethylene glycol and dimethyl carbonate by reacting methanol and ethylene carbonate in the presence of a homogeneous catalyst selected from soluble miscible tertiary phosphines, arsines, and stibines and soluble, miscible bivalent sulphur and selenium compounds.

U.S. Patent No. 4,661,609 relates to a process for the cosynthesis of ethylene glycol and dimethyl carbonate by reacting methanol and ethylene carbonate in the presence of a catalyst selected from zirconium, titanium and tin.

U.S. Patent No. 4,658,041 discloses carbonate compounds which are prepared by equilibrium interchange of carbonate functionality between lower alkyl carbonates, especially dimethyl carbonate, and alkyl halides such as allyl chloride at about 50 ° C to about 250 ° C in the presence of an initiator such as homogeneous or heterogeneous amines, phosphines and ammonium or phosphonium quaternary salts.

U.S. Patent No. 4,349,486 describes a monocarbonate transesterification process comprising contacting in the presence of a base, a beta-fluoroaliphatic carbonate and a compound selected from monohydroxy aliphatic alcohols, phenols and ortho-positioned dihydroxy aromatic compounds.

European Patent Application No. 0110629 relates to a process for the transesterification of a carboxylic or carbonic acid ester which comprises reacting the carboxylic or carbonic acid ester under transesterification conditions with an alcohol in the presence, as catalyst, of either (i) a Group V element-containing Lewis base and an epoxide or (ii) a cyclic amidine. The Lewis base can be an organic compound containing trivalent phosphorus or nitrogen and can be an amine, for example, an amidine. The process can be operated in the liquid phase at a temperature of from 15 to 150 ° C and the catalyst is employed in solution. Preferred catalysts are those where the cyclic amidine forms part of a fused ring system containing six and five membered rings, six and seven membered rings or two six membered rings.

Japanese Kokai Patent No. 54-63023 discloses a method for transesterifying carbonate in which a hydroxy compound and a carbonate are transesterified in the presence of a tin alkoxide.

European Patent Application No. 0119840 describes the preparation of alkylene carbonates and optionally dialkyl carbonates by reaction of an alkylene oxide with carbon dioxide in the presence of an alcohol. In addition there is added to the reaction mixture a trivalent phosphorus containing compound as catalyst. The alkylene carbonate is further transesterified with the alcohol to produce a dialkyl carbonate and an alkylene glycol.

Japanese Kokai Patent No. 63-238043 relates to a method for manufacturing dialkyl carbonate in which a cyclic carbonate and an alcohol are reacted with each other in the presence of a solid high-basicity anion-exchanging material which contains a quaternary ammonium group as an exchange group.

Japanese Kokai Patent No. 55-64550 discloses a method for manufacturing a dialkyl carbonic acid ester in which an alkylene carbonate and aliphatic alcohol are reacted in the presence of a catalyst consisting of a Lewis acid and a nitrogen-containing organic base.

Japanese Kokai Patent No. 56-10144 describes a method for manufacturing dialkyl carbonate in which alkylene carbonate and alcohol are reacted with each other in the presence of a quaternary phosphonium salt catalyst.

Mahe, R. et al., J. Org. Chem., 1989, 54, pp. 1518-1523, relates to a process in which terminal alkynes react with carbon dioxide and secondary amines in the presence of ruthenium complexes to afford vinyl carbamates in one step. The reaction has been performed with phenylacetylene, hexyne and acetylene and with dialkylamines, morpholine, piperidine and pyrrolidine.

Ishii, S. et al., Bull. Chem. Soc. Jpn., 62, 1989, pp. 455-458, discloses a process in which carbon dioxide is reacted with aliphatic amines and ortho esters to form carbamic esters.

Ball, P. et al., C. Mol. Chem., 1984, Vol. 1, pp. 95-108, describes the reaction of urea with aliphatic or cycloaliphatic dihydroxy compounds in the presence of organotin compounds to give polycarbonates.

Izdebski, J. et al., Synthesis, June 1989, pp. 423-425, relates to a method for the preparation of symmetrically and unsymmetrically disubstituted ureas by aminolysis of bis(4-nitrophenyl)carbonate.

U.S. Patent No. 4,327,035 discloses a process for the preparation of carbonic acid esters of monoalcohols or polyalcohols which is characterized in that ureas are reacted with monoalcohols or with polyalcohols in the molar ratio of at least 1:2 in the presence of a catalyst at reaction temperatures between 120 °C and 270 °C.

U.S. Patent No. 3,449,406 describes a process for preparing a carbamate by reacting urea with an alcohol in contact with an aliphatic tertiary polyamine, such as, for example, triethylene diamine or N,N,N',N'-tetramethyl-1,3-butanediamine

U.S. Patent No. 3,627,813 relates to a process in which N,N'-dihydrocarbylureas are converted to the corresponding alkyl N-hydrocarbylcarbamates by heating at a temperature of 60 °C to 200 °C with a dialkylcarbonate in the presence of a base catalyst (tertiary amine preferred).

U.S. Patent No. 3,873,553 discloses a process for preparing N-monosubstituted carbamates wherein a trisubstituted urea is reacted at an elevated temperature with a hydroxy compound and with gaseous hydrogen chloride.

U.S. Patent No. 4,156,784 describes a process in which carbamates are manufactured by reaction of alcohols with urea in the presence of ion exchangers containing nickel.

U.S. Patent No. 4,336,402 relates to a process for the preparation of N-alkyl-monosubstituted carbamic acid esters by reacting an unsubstituted carbamic acid ester and an aliphatic primary amine at a suitable pressure and reaction temperature in the presence of a monohydric aliphatic alcohol and preferably in the presence of a strongly basic tertiary amine as catalyst.

U.S. Patent No. 4,398,036 discloses a process for the preparation of N-monosubstituted carbamates by reacting an aromatic primary amine, urea and a monohydric aliphatic alcohol in the presence of a strongly basic tertiary amine catalyst and optionally an inert solvent.

U.S. Patent No. 4,443,622 describes a process for the manufacture of alkyl carbamates from urea and aliphatic alcohols. The process is carried out under a vacuum, preferably at least 20 inches, and preferably in the presence of magnesium oxide catalyst.

U.S. Patent No. 4,501,915 relates to a process for the preparation of N-alkyl-monosubstituted carbamates by reacting an aliphatic primary amine, urea and a monohydric aliphatic alcohol in the presence of a strongly basic tertiary amine catalyst and optionally an inert solvent.

U.S. Patent No. 4,537,960 discloses a process in which N- and O-substituted carbamates are prepared by reacting urea with an aminopropanol at 140 °C or above.

Enichem Synthesis SpA, Dimethyl Carbonate Product Bulletin, pp. 9-14, describes various transesterification reactions in which dimethyl carbonate is reacted with unsaturated alcohols, phenols, glycols

and diphenols in the presence of small quantities of a basic catalyst, e.g., KOH, NaOH, alcoholates, etc. At page 14, an esterification reaction is described in which dimethyl carbonate reacts with organic acids in the presence of an acid catalyst to produce esters.

Dow Chemical U.S.A., Experimental Ethylene Carbonate XAS-1666.00L Product Bulletin (1982), pp. 6-8, discloses carbamate and imidazolidinone formation through the reaction of ethylene carbonate with aliphatic monoamines and diamines, and also various ester interchange reactions.

Texaco Chemical Company, TEXACAR® Ethylene and Propylene Carbonates Product Bulletin (1987), pp. 22-24, describes the reaction of ethylene carbonate and propylene carbonate with primary and secondary aliphatic amines at low temperature to yield carbamates. It is also stated that ethylene carbonate and propylene carbonate may be used in ester interchange reactions to prepare a number of different polyesters.

## Disclosure of the Invention

This invention relates to a process for preparing carboxylated compounds which comprises contacting an active hydrogen-containing compound with a $CO_2$ synthon in the presence of a mixed metal oxide catalyst or a modified bimetallic or polymetallic catalyst under conditions effective to produce the carboxylated compound.

This invention also relates to a process for preparing carboxylated compounds which comprises contacting one or more $CO_2$ synthons in the presence of a mixed metal oxide catalyst or a modified bimetallic or polymetallic catalyst under conditions effective to produce the carboxylated compound.

This invention further relates to a process for preparing a dialkyl carbonate which comprises contacting an alkylene carbonate with an alkanol in the presence of a mixed metal oxide catalyst or a modified bimetallic or polymetallic catalyst under conditions effective to produce the dialkyl carbonate.

The carboxylated compounds produced in accordance with the processes of this invention are useful for a wide variety of applications such as intermediates for agricultural chemicals, pharmaceutical products and dyestuffs, solvents, fibers and textiles, plastics and resins, gas treating, metal extraction, lubricants, liquid absorbents, fragrances, flavors and the like.

For purposes of this invention, the chemical elements are identified in accordance with the Periodic Table of the Elements, CAS version, Handbook of Chemistry and Physics, 67th Ed., 1986-87, inside cover. Also, for purposes of this invention, Group IIIB metal oxides embraces the lanthanides and actinides. As used herein, the term "oxide" embraces oxides, hydroxides and/or mixtures thereof. Also, as used herein, the term "$CO_2$ synthon" embraces $SO_2$ synthons such as sulfurous acids and sulfurous acid esters. Sulfur analogs of carboxylated compounds, e.g., thiol esters, are also embraced by this invention.

## Detailed Description

As indicated above, this invention relates to a process for preparing carboxylated compounds which comprises contacting an active hydrogen-containing compound with a $CO_2$ synthon in the presence of a mixed metal oxide catalyst or a modified bimetallic or polymetallic catalyst under conditions effective to produce the carboxylated compound.

As also indicated above, this invention relates to a process for preparing carboxylated compounds which comprises contacting one or more $CO_2$ synthons in the presence of a mixed metal oxide catalyst or a modified bimetallic or polymetallic catalyst under conditions effective to produce the carboxylated compound.

As further indicated above, this invention relates to a process for preparing a dialkyl carbonate which comprises contacting an alkylene carbonate with an alkanol in the presence of a mixed metal oxide catalyst or a modified bimetallic or polymetallic catalyst under conditions effective to produce the dialkyl carbonate. In a preferred embodiment, ethylene carbonate and methanol are reacted in the presence of a mixed metal oxide catalyst under conditions effective to produce dimethyl carbonate.

When an active hydrogen-containing compound and $CO_2$ synthon are employed as starting materials, it is believed that a transesterification reaction occurs to provide the desired carboxylated product. The exact reaction mechanism is not fully appreciated but what is appreciated is that an active hydrogen-containing compound starting material and $CO_2$ synthon starting material can be contacted in the presence of a mixed metal oxide catalyst or a modified bimetallic or polymetallic catalyst under conditions described herein to provide a carboxylated compound product. It is also appreciated that one or more $CO_2$ synthons can be contacted in the presence of a mixed metal oxide catalyst or a modified bimetallic or polymetallic catalyst under conditions described herein to provide a carboxylated compound product.

Suitable active hydrogen-containing compound starting materials which can be employed in the processes of this invention include any permissible substituted or unsubstituted active hydrogen-containing organic compound(s). Illustrative active hydrogen-containing compound starting materials useful in this invention include, for example, substituted and unsubstituted alcohols, phenols, carboxylic acids, amines and the like. Preferred active hydrogen-containing compounds include alcohols and phenols. The molar ratio of active hydrogen-containing compound to $CO_2$ synthon is not narrowly critical and can range from about 0.05:1 or less to about 50:1 or greater, preferably from about 0.1:1 to about 10:1.

Suitable active hydrogen-containing compounds include substituted and unsubstituted alcohols (mono-, di- and polyhydric alcohols), phenols, carboxylic acids (mono-, di- and polyacids), and amines (primary and secondary). Other suitable active hydrogen-containing compounds include substituted and unsubstituted thiophenols, mercaptans, amides and the like. Frequently, the organic compounds contain 1 carbon to about 100 or 150 carbons (in the case of polyol polymers) and can contain aliphatic and/or aromatic structures. Most often, the organic compounds are selected from the group of mono-, di- and trihydric alcohols having 1 to about 30 carbon atoms. The organic compounds having active hydrogens can be the product of hydroformylation/hydrogenation reactions.

Suitable alcohols include primary and secondary monohydric alcohols which are straight or branched chain such as methanol, ethanol, propanol, pentanol, hexanol, heptanol, octanol, nonanol, decanol, undecanol, dodecanol, tridecanol, tetradecanol, pentadecanol, hexadecanol, octadecanol, isopropyl alcohol, 2-ethylhexanol, sec-butanol, isobutanol, 2-pentanol, 3-pentanol and isodecanol. Particularly suitable alcohols are linear and branched primary alcohols (including mixtures) such as produced by the "Oxo" reaction of $C_3$ to $C_{20}$ olefins. The alcohols may also be cycloaliphatic such as cyclopentanol, cyclohexanol, cycloheptanol, cyclooctanol, as well as aromatic substituted aliphatic alcohols such as benzyl alcohol, phenylethyl alcohol, and phenylpropyl alcohol. Other aliphatic structures include 2-methoxyethanol and the like.

Phenols include alkylphenols of up to 30 carbons such as p-methylphenol, p-ethylphenol, p-butylphenol, p-heptylphenol, p-nonylphenol, dinonylphenol and p-decylphenol. The aromatic radicals may contain other substituents such as halide atoms.

Alcohols (polyols) having 2 or more hydroxyl groups, e.g., about two to six hydroxyl groups and have 2 to 30 carbons, include glycols such as ethylene glycol, propylene glycol, butylene glycol, pentylene glycol, hexylene glycol, neopentylene glycol, decylene glycol, diethylene glycol, triethylene glycol and dipropylene glycol. Other polyols include glycerine, 1,3-propanediol, pentaerythritol, galactitol, sorbitol, mannitol, erythritol, trimethylolethane and trimethylolpropane.

Carboxylic acids include formic acid, oxalic acid, acetic acid, propionic acid, butyric acid, valeric acid, caproic acid, caprylic acid, capric acid, lauric acid, myristic acid, palmitic acid, stearic acid, oleic acid, linoleic acid and linolenic acid. Other carboxylic acids include benzoic acid, cyclohexane carboxylic acid, phenylacetic acid, toluic acid, chlorobenzoic acid, bromobenzoic acid, nitrobenzoic acid, phthalic acid, isophthalic acid, terephthalic acid, salicylic acid, hydroxybenzoic acid, anthranilic acid, aminobenzoic acid, methoxybenzoic acid and the like.

Amines include methylamine, dimethylamine, ethylamine, diethylamine, n-propylamine, di-n-propylamine, isopropylamine, n-butylamine, isobutylamine, cyclohexylamine, piperazine, benzylamine, phenylethylamine, monoethanolamine, diethanolamine, aminoethylethanolamine, ethylenediamine, diethylenetriamine, triethylenetetramine, tetraethylenepentamine, pentaethylenehexamine, tetramethylenediamine and hexamethylenediamine. Other amines include aniline, methylaniline, toluidine, anisidine, chloroaniline, bromoaniline, nitroaniline, diphenylamines, phenylenediamine, benzidine, aminobenzoic acid, sulfanilic acid and sulfanilamide. Still other amines include acetanilide, benzanilide, acetotoluidide, nitroacetanilide and the like.

Preferred active hydrogen-containing compound starting materials which can be employed in the processes of this invention include any permissible active hydrogen-containing organic compounds such as those embraced by the formula $R(OH)_m$ wherein R is the residue of an organic compound and m is a value which satisfies the valencies of R, preferably m is a value of from 1 to about 6, more preferably m is a value of from 1 to about 4. Preferred active hydrogen-containing compound starting materials include monohydric, dihydric and polyhydric alcohols and alkanolamines. Illustrative active hydrogen-containing compound starting materials useful in this invention include, for example, ethylene glycol, diethylene glycol, triethylene glycol, tetraethylene glycol, pentaethylene glycol, hexaethylene glycol, polyethylene glycols, $C_1$ - $C_{20}$ alcohols including mixtures, 2-(2-methoxyethoxy)ethanol, 2-(2-butoxyethoxy)ethanol, 2-methoxyethanol, poly(oxyalkylene)glycols such as CARBOWAX® poly(oxyethylene)glycols, alkoxy and allyloxy poly(oxyalkylene)glycols, alkoxy and allyloxy poly(oxyethylene)(oxypropylene)glycols such as UCON® fluid and lubricant materials, aminoethylethanolamine and the like.

Suitable $CO_2$ synthon starting materials which can be employed in the processes of this invention

include any permissible substituted or unsubstituted carboxyl-containing compound(s) or carbonyl-containing compound(s) which are capable of reacting with an active hydrogen-containing compound under the process conditions described herein, such as those embraced by the formulae $R_1C(O)R_2$ or $R_1S(O)R_2$ wherein $R_1$ is hydrogen, halogen, amino, hydroxyl or the residue of an organic compound, and $R_2$ is amino, hydroxyl or the residue of an organic compound. Illustrative $CO_2$ synthons include, for example, substituted and unsubstituted carbonates, chlorocarbonates, carbonic acids, carbamates, carbamic acids, oxalates, 2-oxazolidinones, ureas, esters, phosgene, chloroformates, carbon dioxide, orthocarboxylates, sulfurous acids, sulfurous acid esters and the like. For purposes of this invention, carbon monoxide is also considered a $CO_2$ synthon for appropriate oxidative carbonylation reactions. Preferred $CO_2$ synthons include, for example, diethyl carbonate, ethylene carbonate, propylene carbonate, dimethyl carbonate, 2-oxazolidinone, ethylene sulfite and the like. The use of $CO_2$ synthons prepared in situ such as the reaction of ethylene carbonate and monoethanolamine to give 2-oxazolidinone is encompassed within the scope of this invention.

As indicated above, $R_1$ and $R_2$ can be the residue of an organic compound. Illustrative residues of organic compounds include, for example, alkyl, aryl, alkylamino, arylamino, cycloalkyl, heterocycloalkyl, alkyloxy, aryloxy, cycloalkyloxy, heterocycloalkyloxy, alkyloxycarbonyl, aryloxycarbonyl, cycloalkyloxycarbonyl, heterocycloalkyloxycarbonyl, hydroxycarbonyl and the like. Additionaly, for purposes of defining the $CO_2$ synthon by the formulae above, the $R_1$ and $R_2$ substituents together can complete a cycloalkyl ring or a heterocycloalkyl ring which can be substituted or unsubstituted. The $R_1C(O)R_2$ formula is also contemplated to embrace carbon dioxide and carbon monoxide.

Suitable catalysts which can be employed in the processes of this invention include two or more metal oxides. A magnesium:aluminum mixed metal oxide is a preferred mixed metal oxide catalyst as more fully described below. Both homogeneous and heterogeneous catalysts can be employed in the reaction. The amount of catalyst used in the reaction is not narrowly critical and is dependent on whether the reaction is conducted batchwise or continuously. If batchwise, the catalyst employed can range from about 0.01 weight percent or less to about 10 weight percent or greater of the total weight of the starting materials. If continuously, generally a fixed bed is employed.

Suitable catalysts for use in the processes of this invention comprise mixed metal oxides containing two or more metal oxides. Illustrative of such mixed metal oxides include, for example, two or more of the following: Group IA metal oxides, Group IIA metal oxides, Group IIIB metal oxides (including lanthanides and actinides), Group IVB metal oxides, Group VB metal oxides, Group VIB metal oxides, Group VIIB metal oxides, Group VIII metal oxides, Group IB metal oxides, Group IIB metal oxides, Group IIIA metal oxides, Group IVA metal oxides, Group VA metal oxides or Group VIA metal oxides. Preferred mixed metal oxides are amphoteric or basic. Preferred mixed metal oxides which may be utilized as catalysts include, for example, two or more oxides of magnesium, aluminum, calcium, strontium, gallium, beryllium, barium, scandium, yttrium, lanthanum, cerium, gadolinium, terbium, dysprosium, holmium, erbium, thulium, lutetium, ytterbium, niobium, tantalum, chromium, molybdenum, tungsten, titanium, zirconium, hafnium, vanadium, iron, cobalt, nickel, zinc, silver, cadmium, boron, indium, silicon, germanium, tin, lead, arsenic, antimony and bismuth.

Group IIA metal oxides such as magnesium oxide and calcium oxide and Group IIIA metal oxides such as aluminum oxide and gallium oxide are preferred mixed metal oxides for use in this invention. For mixed metal oxides in which at least one of the metals is magnesium, suitable metals in association with magnesium may include, for example, one or more of the following: Group IIIA metals such as boron, aluminum, gallium and indium, Group IIIB metals such as scandium, yttrium and lanthanum including the lanthanides, Group VB metals such as niobium and tantalum, Group VIB metals such as chromium, molybdenum and tungsten, Group VIII metals such as iron, cobalt and nickel, Group IIB metals such as zinc and cadmium, Group IVA metals such as silicon, germanium, tin and lead, Group VA metals such as arsenic, antimony and bismuth, and Group IVB metals such as zirconium and hafnium. For mixed metal oxides in which at least one of the metals is calcium, suitable metals in association with calcium may include, for example, one or more of the following: Group IIIA metals such as boron, aluminum, gallium and indium, Group IVA metals such as silicon, germanium, tin and lead, Group VB metals such as niobium and tantalum, and Group VIB metals such as chromium, molybdenum and tungsten.

Illustrative of mixed metal oxides which may be used as catalysts include, for example, $MgO-Al_2O_3$, $MgO-SiO_2$, MgO-CdO, $MgO-Bi_2O_3$, $MgO-Sb_2O_5$,$MgO-SnO_2$, $Mgo-ZrO_2$, MgO-BeO, $MgO-TiO_2$, MgO-CaO, MgO-SrO, MgO-ZnO, $MgO-Ga_2O_3$, $MgO-Y_2O_3$, $Mgo-La_2O_3$, $MgO-MoO_3$, $MgO-Mn_2O_3$, $MgO-Fe_2O_3$, $MgO-Co_3O_4$, $MgO-WO_3$, $MgO-V_2O_5$, $MgO-Cr_2O_3$, $MgO-ThO_2$, $MgO-Na_2O$, MgO-BaO, MgO-CaO, $MgO-HfO_2$, $MgO-Li_2O$, $MgO-Nb_2O_5$, $MgO-Ta_2O_5$, $MgO-Gd_2O_3$, $MgO-Lu_2O_3$, $MgO-Yb_2O_3$, $MgO-CeO_2$, $MgO-Sc_2O_3$, MgO-PbO, MgO-NiO, MgO-CuO, MgO-CoO, $MgO-B_2O_3$, $CaO-SiO_2$, $CaO-Al_2O_3$, CaO-SnO, CaO-PbO, CaO-

$Nb_2O_5$, CaO-$Ta_2O_5$, CaO-$Cr_2O_3$, CaO-$MoO_3$, CaO-$WO_3$, CaO-$TiO_2$, CaO-$HfO_2$, MgO-$SiO_2$-$Al_2O_3$, MgO-$SiO_2$-ZnO, MgO-$SiO_2$-$ZrO_2$, MgO-$SiO_2$-CuO, MgO-$SiO_2$-CaO, MgO-$SiO_2$-$Fe_2O_3$, MgO-$SiO_2$-$B_2O_3$, MgO-$SiO_2$-$WO_3$, MgO-$SiO_2$-$Na_2O$, MgO-$SiO_2$-$Ga_2O_3$, MgO-$SiO_2$-$La_2O_3$, MgO-$SiO_2$-$Nb_2O_5$, MgO-$SiO_2$-$Mn_2O_3$, MgO-$SiO_2$-$Co_3O_4$,MgO-$SiO_2$-NiO, MgO-$SiO_2$-PbO, MgO-$SiO_2$-$Bi_2O_3$, MgO-$Al_2O_3$-ZnO, MgO-$Al_2O_3$-$ZrO_2$, MgO-$Al_2O_3$-$Fe_2O_3$,MgO-$Al_2O_3$-$WO_3$, MgO-$Al_2O_3$-$La_2O_3$, MgO-$Al_2O_3$-$Co_3O_4$, CaO-$SiO_2$-$Al_2O_3$, CaO-$SiO_2$-SnO, CaO-$SiO_2$-$Nb_2O_5$, CaO-$SiO_2$-$WO_3$, CaO-$SiO_2$-$TiO_2$, CaO-$SiO_2$-$MoO_3$, CaO-$SiO_2$-$HfO_2$, CaO-$SiO_2$-$Ta_2O_5$, CaO-$Al_2O_3$-$SiO_2$, CaO-$Al_2O_3$-PbO, CaO-$Al_2O_3$-$Nb_2O_5$, CaO-$Al_2O_3$-$WO_3$,CaO-$Al_2O_3$-$TiO_2$, CaO-$Al_2O_3$-$MoO_3$, CaO-$HfO_2$-$Al_2O_3$,CaO-$HfO_2$-$TiO_2$, and the like. Other suitable mixed metal oxides embraced within the scope of this invention are disclosed by Tanabe et al., Bulletin of the Chemical Society of Japan, Vol. 47(5), pp. 1064-1066 (1974).

The mixed metal oxides described herein which can be used as catalysts may contribute to product selectivity and/or catalytic activity of the reaction and/or stability of the catalyst. As discussed hereinafter, the catalyst employed in this invention may also contain support(s), binding agent(s) or other additives to stabilize or otherwise help in the manufacture of the catalyst.

The catalysts which comprise two or more metal oxides may be prepared in a wide variety of ways. For example, the two or more metal oxides can be provided from metal salts which can either be heated or precipitated to form the mixed metal oxides. Also, two or more metal oxides may be provided as a partial condensate on a support, such as a silica or alpha, beta or gamma alumina, silicon carbide, and the like, and then condensed by heating to effect polymerization to the desired oxide form. The two or more metal oxides may be condensed from hydrolyzable monomers to the desired oxides, indeed, to form oxide powders which can thereafter be compressed in the presence of a condensation catalyst to form pellets and larger structures of the mixed metal oxide catalyst. A blend of the powders and condensation catalyst can be made into a shapeable paste which can be extruded and cut into pellets according to conventional procedures. The extrudate may thereafter be fired to cure the condensation catalyst and fix the structure. The cut extrudate may be blended with a support material such as those characterized above, and the blend fired to fuse the mixed metal oxide catalyst to the support.

In an embodiment of this invention, a magnesium salt, e.g., magnesium nitrate, and an aluminum salt, e.g., aluminum nitrate, are precipitated using ammonium hydroxide. The material is then washed with deionized water and calcined at a temperature of from about 350°C to about 450°C to afford the desired magnesium:aluminum mixed metal oxide catalyst.

In another embodiment, a magnesium oxide, e.g., magnesium carbonate hydroxide pentahydrate, and an aluminum oxide, e.g., aluminum hydroxide hydrate, are added to deionized water and thoroughly mixed to form a paste. The paste is then calcined at a temperature of from about 350°C to about 450°C to afford the desired magnesium:aluminum mixed metal oxide catalyst.

A preferred catalyst structure comprises a Group IIA and IIIA mixed metal oxide having a surface area of at least about 100 $m^2$/gm which may or may not be bonded to a support material. The catalysts on a support preferably have a surface area greater than about 20 $m^2$/gm to as high as about 260 $m^2$/gm, or greater depending upon which metal oxides are employed. In the case of magnesium:aluminum oxides, the surface area can be greater than about 50 $m^2$/gm to as high as about 260 $m^2$/gm, more preferably, greater than about 100 $m^2$/gm to as high as about 260 $m^2$/gm, determined according to the single point $N_2$ method.

The term "support," as used herein and in the claims, means a solid structure which does not adversely affect the catalytic properties of the catalyst and is at least as stable as the catalyst to the reaction medium. The support can function as a catalyst independent of the mixed metal oxide catalyst used herein, although it may have lower catalytic activity to the reaction. The support may act in concert with the catalyst to moderate the reaction. Some supports may contribute to the selectivity of the reaction. The catalyst structure can comprise from about 2 to about 60 percent by weight or greater of the support, more preferably from about 10 to about 50 percent by weight of the support, the remainder being the weight of the mixed metal oxides. Included in the weight of the support is the weight of any binding agent such as phosphates, sulfates, silicates, fluorides, and the like, and any other additive provided to stabilize or otherwise help in the manufacture of the catalyst. The support may be particles as large or larger than the catalyst component and "glued" to the catalyst by virtue of a binding medium.

The support may constitute a separate phase in the process of extruding the catalytic structure. In this embodiment, the support forming material, preferably as a paste is blended with a paste of the catalyst or a partial condensate thereof. The paste may comprise the oxide forms of the support and the catalyst, each blended with water, and/or binding agents. The extrudate of the blend is passed through a multiorificed die and chopped into pellets of the desired sizes. The particles may be doughnut shaped, spherical, and the like. Then the particles are calcined to dry them and complete any condensation reaction in the support and/or the mixed metal oxide catalyst.

A preferred group of mixed metal oxide catalysts for use in this invention include materials having the formula:

$$M_x^{2+} Q_y^{3+} (OH)_{2x+3y-nz} A_z^{n-} \cdot a\ H_2O \qquad (I)$$

wherein M is at least one divalent metal cation; Q is at least one trivalent metal cation; and A is at least one anion providing a valence ($n^-$), wherein n is at least 1, e.g., between 1 and 4 and most often between 1 and 3, and wherein a is a positive number, M, Q, and A are provided in a proportion such that x/y is a number equal to or greater than 1, z has a value greater than zero and 2x + 3y-nz is a positive number. M, Q and A may be selected to provide a layered structure. Preferably, x/y is in the range of 1 to 12, more preferably x/y is in the range of 1 to 6 and most preferably is in the range of 1 to 4. Preferably, z has a value such that x/z is between n and 12n, more preferably between n and 6n and most preferably between n and 4n.

Suitable divalent metal cations, M, broadly include elements selected from the Transition elements and Groups IIA and IVA of the Periodic Table as well as certain Group IIIB elements. As specific examples can be mentioned magnesium, calcium, titanium, vanadium, chromium, manganese, iron, cobalt, nickel, palladium, platinum, copper, zinc, cadmium, mercury, tin and lead. Divalent metal cations which are particularly suitable are magnesium, nickel, cobalt, zinc, calcium, strontium and copper. Suitable trivalent metal cations, Q, broadly include elements selected from the Transition elements and Groups IIIA and VA of the Periodic Table as well as certain Group IIIB elements. As specific examples can be mentioned aluminum, antimony, titanium, scandium, bismuth, vanadium, yttrium, chromium, iron, manganese, cobalt, ruthenium, nickel, gold, gallium, thallium, and cerium. Trivalent metal cations which are particularly suitable can be selected from aluminum, boron, gallium and lanthanum.

The composition of formula (I) also can include a wide range of anions, A. Any anion or combination of anions which can balance the charge of the cations can be used. Suitable anions include inter alia, halides (such as chloride, fluoride, bromide, and iodide), nitrite, nitrate, sulfite, sulfate, sulfonate, carbonate, chromate, cyanate, phosphite, phosphate, molybdocyanate, bicarbonate, hydroxide, arsenate, chlorate, ferrocyanide, borate, cyanide, cyanaurate, cyanaurite, ferricyanide, selenate, tellurate, bisulfate, as well as organic anions such as oxalate, acetate, hexanoate, sebacate, formate, benzoate, malonate, lactate, oleate, salicylate, stearate, citrate, tartrate, maleate, and the like. The class of metalate anions described in U.S. Patent No. 4,667,045, including metavanadate, orthovanadate, molybdate, tungstate, hydrogen pyrovanadate and pyrovanadate, also are suitable as anion A. Anions suitable for use in combination with the metal cations previously identified as being particularly suitable are carbonate, halide, phosphate, chromate, sulfate, hydroxide, oxalate, acetate, nitrate, hexanoate, sebacate, vanadate, molybdate, tungstate and ferrocyanate.

The foregoing lists of suitable divalent and trivalent cations and suitable anions are meant to be illustrative and not exclusive. Those skilled in the art will recognize that other cations and anions can be used provided that the specific type of cations and their relative amounts (x/y ratio) and the specific type of anions and their relative amount result in a mixed metal oxide composition.

Included in the materials identified above are those based on exchangeable anionic clay minerals. For example, compositions of formula (I) wherein M is magnesium and Q is aluminum are related to hydrotalcites, while compositions in which M is nickel and A is aluminum are related to takovites. In fact, mixed metal oxides prepared using magnesium, nickel or cobalt as the divalent cation and aluminum as the trivalent cation exhibit the typical X-ray diffraction pattern of a hydrotalcite.

In another preferred aspect, the processes of this invention can utilize mixed metal oxide catalyst compositions prepared by calcining at an elevated temperature compositions according to formula (I). Suitable calcined compositions have the general formula:

$$M_x^{2+} Q_y^{3+} (O)_{(2x+3y-nz)/2} D_z^{n-} \qquad (II)$$

wherein M, Q, x, y, z and n have the same meanings defined above in connection with formula (I), and D is at least one nonvolatile anion. Nonvolatile anions may include, inter alia, halides, nitrates, phosphites, phosphate, vanadate, molybdate, tungstate, sulfite, sulfate, chromate, arsenate, borate, chlorate and the like. This list is illustrative and not exclusive.

Heat treating the formula (I) compositions to prepare the calcined mixed metal oxide compositions of

formula (II) can be done, for example, at a temperature in the range of 200 ° C to 800 ° C for a period of time of about 12 to 24 hours under an inert atmosphere such as nitrogen or in appropriate cases under an oxidizing atmosphere such as air.

Calcination of the mixed metal oxide composition dehydrates the composition and converts at least partially the metal hydroxides to metal oxides. Any nonvolatile anions may be present in the calcined material.

Provided the calcination temperature is not excessive, the mixed metal oxide can be rehydrated to the mixed metal hydroxide with water. Generally, the mixed metal oxide can be restored readily if the calcination temperature does not exceed about 600 ° C. Mixed metal oxides which are calcined under more severe conditions are not easily rehydrated and lower surface area materials are obtained.

Certain compositions falling within formula (I), such as hydrotalcite, which comprises a magnesium-aluminum hydroxide carbonate, and takovite, which comprises a nickel-aluminum hydroxide carbonate, are naturally occurring compositions. However, such compounds, as well as their related compositions, also can be prepared synthetically from inexpensive starting materials using well-known coprecipitation techniques. Procedures for direct synthesis of such materials are described in Itaya et al., Inorg. Chem. (1987) 26:624-626; Taylor, R.M., Clay Minerals (1984) 19:591-603; Reichle, U.S. Patent No. 4,476,324; Bish, D.L., Bull. Mineral (1980), 103:170-175 and Miyata et al., Clays and Clay Minerals (1977), 25:14-18. Using direct synthesis one has the ability to vary within wide limits the $M^{+2}/Q^{+3}$ atomic ratio as well as the anion.

For example, a composition of formula (I) where $M^{+2}$ is nickel or magnesium, $Q^{+3}$ is aluminum and $A^{n-}$ is carbonate can be prepared by adding, as aqueous solutions, (a) a mixture of nitrates, sulfates or chlorides of nickel or magnesium and aluminum in a desired atomic ratio of nickel or magnesium to aluminum, e.g. 6 atoms of nickel as nickel chloride to 2 atoms of aluminum as aluminum chloride, to (b) an aqueous solution of a stoichiometric amount of sodium hydroxide and a water soluble salt of the desired anion, e.g., sodium carbonate. The two solutions are mixed at a temperature of about 25 ° C to 35 ° C with vigorous stirring over a several-hour period to produce a slurry. The slurry then is heated for about 18 hours at a temperature within the range of about 50 ° C to 200 ° C (preferably between about 60 ° C to 75 ° C) in order to control crystallization and the ultimate particle size of the resulting crystals. After filtering, and thorough washing and drying, the solids are recovered, typically as a powder.

As noted above, this procedure can be adapted to a wide variety of cations, cation atomic ratios and anion substitutions. For example, water soluble salts of divalent magnesium, cobalt, zinc, copper, iron and calcium can be substituted for the nickel chloride illustrated above, while water soluble salts of trivalent gallium and lanthanum can replace the aluminum chloride. A wide variety of other combinations also will be apparent to those skilled in the art. Generally, the rate of metal ion addition to the aqueous caustic/anion solution is not critical and can be varied widely. For example, a preferred preparation method is described in Schaper, H. et al., Applied Catalysis, 54, 1989, 79-90, the disclosure of which is incorporated herein by reference. The reaction temperature also is not critical, although the temperature during the reaction preferably is kept below about 100 ° C. An important feature of the procedure is the use of efficient agitation during the mixing procedure to avoid the formation of undesired by-products.

Loading of an anion A or D into the mixed metal oxide compositions is influenced by a variety of factors including (i) the amount of anion used in the preparation relative to the metal cations, (ii) the atomic ratio of the metal cations (x/y) in the preparation procedure, (iii) the size of the cations and anions and (iv) the preparation procedure. As used herein, "loading" is defined as the amount of available valences provided by a desired anion A or D expressed as a percentage of the total available valences for anion A or D. For example, carbonate loading in a hydrotalcite-type catalyst can be maximized by (i) using an excess (e.g., a greater than 3:1 molar ratio) of sodium carbonate to aluminum chloride during catalyst preparation and (2) adjusting the atomic ratio of magnesium to aluminum cations to about 2:1. Mixed metal oxide compositions suitable as catalysts also can be prepared from the native or synthetic hydrotalcite-type compositions by ion exchange. For example, hydrotalcite can be treated at ambient conditions with 0.01N phosphoric acid for about 18 hours to replace the carbonate anion with phosphate anion. A halide analog of hydrotalcite prepared directly or by anion-exchange could be contacted with molybdic acid or a water soluble salt thereof, or with a water soluble salt of tungstic acid or vanadic acid in order to substitute the transition metal anion for the halide anion in the catalyst structure thereby to produce a mixed metal oxide composition of formula (I). Other ion exchanges will be apparent to those skilled in the art.

Calcined mixed metal oxide compositions may exhibit a higher level of selectivity/activity than uncalcined compositions. If a calcined mixed metal oxide catalyst composition experiences any decline in selectivity, it can be regenerated by a heat treatment in the presence of air to restore at least a portion of its initial level of selectivity/activity enhancement and reused. Conditions discussed above for calcining the hydrated mixed metal oxide compositions are suitable for regenerating compositions which have experienced a decline in activity.

Catalysts having the formulas (I) and (II) above wherein M is at least one of magnesium and calcium, Q is aluminum or gallium, A is at least one of carbonate, bicarbonate, phosphate, sulfate and nitrate, x/y is between 1 and 20, z has a value which satisfies the relationship: x/z is between n and 12n, and a is a positive number, are generally preferred for vapor phase decarboxylation due to their combination of activity (conversion of precursor) and selectivity. A preferred process involves a vapor phase process using mixed metal oxide catalyst wherein $M^{2+}$ is magnesium, $Q^{3+}$ is aluminum, $A^{n-}$ is carbonate, x/y is about 1, and z is about 1.

A group of preferred mixed metal oxide catalyst compositions which can be employed in the processes of this invention is disclosed in copending U.S. Patent Application Serial No. 125,134, filed November 25, 1987, the disclosure of which is incorporated herein by reference.

The modified bimetallic or polymetallic catalysts useful in this invention comprise one or more metal-containing compounds which are modified with an organic or inorganic oxyacid having a divalent or polyvalent anion such as sulfuric acid, phosphoric acid, carbonic acid, pyrosulfuric acid and the like, or by metal salts of organic or inorganic oxyacids having divalent or polyvalent anions such as aluminum sulfate, zinc sulfate, zinc phosphate and the like or mixtures thereof. These modified catalysts are believed to have complex structures which are probably comprised of a mixture of species, certain of which may not even be catalytically active. Those species which are catalytically active are believed to have structures of the type depicted by the following formula:

$$[R_4\text{-}X_1\text{-}M_1]_f\text{-}Y_1\text{-}[M_3\text{-}Y_2]_j\text{-}[M_2\text{-}X_2\text{-}R_5]_g \qquad \text{(III)}$$

wherein:

$R_4$ and $R_5$ are independently hydrogen or the residue of an organic compound having at least one active hydrogen;

$X_1$ and $X_2$ are independently oxygen, sulfur or nitrogen;

$M_1$, $M_2$ and $M_3$ are independently a divalent or polyvalent metal;

$Y_1$ and $Y_2$ are independently a divalent or polyvalent oxyacid anion of valence 2 to 6, oxygen, sulfur or nitrogen provided at least one of $Y_1$ and $Y_2$ is a divalent or polyvalent oxyacid anion of valence 2 to 6;

j is an integer having a value of from 0 to about 100; and

f and g are integers having a value such that the sum f + g is equal to the valence of $Y_1$ when j is a value of 0, and f and g are integers having a value such that the sum f + g is equal to the valence of $Y_1$ plus $[M_3\text{-}Y_2]_j$ when j is a value other than 0. It is understood that the above catalyst formula is speculation only.

The modifier of the catalyst is a divalent or polyvalent acid or a divalent or polyvalent metal salt of an oxyacid or mixtures thereof and contains at least one, most often at least about 2, oxygen atoms that are conventionally depicted as double bonded to the nucleus atom. Such acids and metal salts include, for example, sulfuric and phosphoric acid and the sulfates and phosphates of zirconium, zinc and aluminum.

The types of divalent and polyvalent anions of oxacids and metal salts of oxyacids suitable for use in this invention, e.g., $Y_1$ and $Y_2$, include by way of example only, sulfates, e.g., $SO_4^{-2}$ , phosphates, e.g., $PO_4^{-3}$, manganates, e.g., $MnO_4^{-2}$, titanates, e.g., $TiO_3^{-2}$, tantalates, e.g., $Ta_2O_6^{-2}$, molybdates, e.g., $MoO_4^{-2}$, vanadates, e.g., $V_2O_4^{-2}$, chromates, e.g., $CrO_4^{-2}$, zirconates, e.g., $ZrO_3^{-2}$, polyphosphates and the like.

Illustrative of metals which may be included in the divalent or polyvalent metal salt modifier and also in the bimetallic and polymetallic catalysts include calcium, magnesium, strontium, barium, lanthanum, titanium, zirconium, hafnium, vanadium, niobium, tantalum, chromium, molybdenum, tungsten, manganese, iron, cobalt, nickel, copper, zinc, cadmium, mercury, boron, aluminum, gallium, indium, thallium, carbon, silicon, germanium, tin, lead, phosphorus, arsenic, antimony, sulfur, selenium and tellurium. Calcium or calcium in combination with a bivalent or polyvalent metal are preferred metals for the modified bimetallic and polymetallic catalysts.

In general, at the time of modification, the catalyst precursor composition may be represented by the following formula:

$$[R_4\text{-}X_1\text{-}M_1]_f\text{-}X_3\text{-}[M_3\text{-}X_4]_j\text{-}[M_2\text{-}X_2\text{-}R_5]g \qquad \text{(IV)}$$

wherein $R_4$, $R_5$, $X_1$, $X_2$, $M_1$, $M_2$, $M_3$ and j are as defined hereinabove for formula (III), $X_3$ and $X_4$ are independently oxygen, sulfur or nitrogen, and f and g are integers having a value such that the sum f + g is equal to the valence of $X_3$ when j is a value of 0, and f and g are integers having a value such that the sum

f + g is equal to the valence of $X_3$ plus $[M_3\text{-}X_4]_j$ when j is a value other than 0. $R_4$ and $R_5$ independently may also contain double bonded oxygen (the organic compound was a carboxylic acid), heteroatom such as oxygen, sulfur, nitrogen, and phosphorous (e.g., the organic compound was a glycol, polyamine, ether of a glycol or the like). Frequently, $R_4$ and $R_5$ may comprise 1 to 20 carbons. It is understood that the above catalyst precursor formula is speculation only.

For purposes of this invention including the claims hereinafter, it is understood that the catalyst precursor formula shall be inclusive of polyvalency requirements for $M_1$, $M_2$ and $M_3$ and that such polyvalency requirements are appropriately satisfied in the formula. It is also understood that any polyvalency requirements of $M_3$ may be satisfied by $R_4\text{-}X_1\text{-}$ or $R_5\text{-}X_2\text{-}$.

The preparation of modified bimetallic and polymetallic catalysts is characterized by a considerable degree of operational latitude. A preferred preparation involves the following steps:

Step 1 - Reaction of a divalent or polyvalent metal or a metal-containing compound with a suitable first organic compound having at least one active hydrogen to produce a first divalent or polyvalent metal-containing composition.

Step 2 - Reaction of a divalent or polyvalent metal or other suitable source of divalent or polyvalent metal with a suitable second organic compound containing at least one active hydrogen to produce a second divalent or polyvalent metal-containing composition.

Step 3 - Reaction of the first divalent or polyvalent metal-containing composition with the second divalent or polyvalent metal-containing composition to produce the catalyst precursor composition.

Step 4 - Reaction of the catalyst precursor composition with a suitable third organic compound having at least one active hydrogen optionally to effect exchange of the first and/or second organic compound-derived organic radicals for the third organic compound-derived organic radicals.

During or following the exchange reactions of step 4, the first and/or second organic compounds, which preferably are substantially more volatile than the third organic compound, are removed from the system by distillation. At the conclusion of this operation, the unmodified version of the catalyst is obtained in the form of a residue-free slurry in the third organic compound.

In the preparation of the intermediate unmodified form of the bimetallic or polymetallic catalyst, steps 1 and 2 may be combined into one operation. The first and second divalent or polyvalent metal-containing compositions prepared in steps 1 and 2 may be the same compositions, thereby omitting step 2. Additionally, steps 1 and 4 above may be combined into one operation wherein the divalent or polyvalent metal or the divalent or polyvalent metal-containing compound is reacted with a mixture of a suitable first and third organic compounds. Alternatively, step 2 may be omitted and a divalent or polyvalent metal salt of an oxyacid used in step 5 below. The procedure of combining steps 1 and 4 may be employed because it tends to minimize color build-up in the catalyst slurry. From the standpoint of the final product characteristics, both procedures are equally acceptable. Modified processes wherein the first organic compound is fed into a slurry of the third organic compound and the divalent or polyvalent metal base or the third organic compound is fed into a slurry (or, in some cases, a solution) of the divalent or polyvalent metal base in the first organic compound are also operationally viable, although their use offers no perceived advantage over the batch-charging version.

The preparation of the modified catalyst involves a fifth processing step which, like that of steps 1 through 4, is a distinct step in terms of the chemistry which takes place.

Step 5 - Treatment of the slurry of unmodified catalyst in the third organic compound with a deficiency of some appropriate modifier such as a divalent or polyvalent oxyacid or a divalent or polyvalent metal salt of an oxyacid or mixtures thereof.

This step provides a highly-active, modified bimetallic or polymetallic catalyst in the form of a slurry in the third organic compound.

Preferred bimetallic and polymetallic catalysts for use in this invention are described in U.S. Patent No. 4,820,673, U.S. Patent No. 4,754,075 U.S. Patent No. 4,886,917, and copending U.S. Patent Application Serial No. 298,897, filed January 18, 1989, all of which are incorporated herein by reference. Other preferred bimetallic and polymetallic catalysts for use in this invention are described in copending U.S. Patent Application Serial Nos. 251,434, 251,430, 251,433, 251,432, 251,436 and 251,431, all filed September 30, 1988, and all incorporated herein by reference.

The processes of this invention can be conducted over a wide range of pressures ranging from atmospheric or subatmospheric pressures to superatmospheric pressures. However, the use of very high pressures has not been observed to confer any significant advantages but increases equipment costs. Further, it is preferable to conduct the reaction at reduced pressures of from about 1 mm Hg to less than

about 760 mm Hg. The reaction is preferably effected in the liquid or vapor or supercritical liquid states or mixtures thereof.

The temperature of the processes of this invention may be as low as about ambient temperature to about 300 ° C. Preferably, the reaction temperature ranges from about 50 ° C to about 200 ° C, and most preferably from about 60 ° C to about 120 ° C.

Suitable carboxylated compounds prepared by the processes of this invention include any permissible substituted and unsubstituted carboxylated compounds, including substituted and unsubstituted esters, carbamic acid esters and carbonic acid esters, such as those embraced by the formulae $ROC(O)R_1$, $ROC(O)R_2$, $ROC(O)OR_1$, $ROC(O)OR_2$, $RN(C(O)OR_1)R$ or $RN(C(O)OR_2)R$ wherein R, $R_1$ and $R_2$ are as defined above. It is understood that the R and $R_1$ substituents together, the R and $R_2$ substituents together, and the R substituents together can complete a cycloalkyl ring or a heterocycloalkyl ring which can be substituted or unsubstituted. Illustrative carboxylated compounds include, for example, dimethyl carbonate, diethyl carbonate, bis(2-methoxyethyl)carbonate, bis[2-(2-methoxyethoxy)ethyl]carbonate, diallyl carbonate, bis(2-butoxyethyl)carbonate and the like.

Illustrative of suitable carboxylated compounds which can be prepared by the processes of this invention include those permissible carboxylated compounds, including any permissible derivatives of described carboxylated compounds, which are described in Kirk-Othmer, Encyclopedia of Chemical Technology, Third Edition, 1984, the pertinent portions of which are incorporated herein by reference.

The carboxylated compounds prepared by the processes of this invention may undergo one or more additional transesterifications. For example, an active hydrogen-containing compound different from the active hydrogen-containing compound starting material may be reacted with the prepared carboxylated compound under conditions effective to prepare a different carboxylated compound. Suitable active hydrogen-containing compounds include those embraced by the formula $R_3OH$ wherein $R_3$ is the residue of an organic compound.

The carboxylated compounds prepared in accordance with this invention can be either symmetrical or unsymmetrical. For the preparation of carboxylated compounds having a symmetrical configuration, it is preferred to use an alkylene carbonate, e.g., ethylene carbonate or propylene carbonate, as the $CO_2$ synthon starting material or a molar excess of any active hydrogen-containing compound starting material with any $CO_2$ synthon starting material, e.g., a molar ratio of active hydrogen-containing compound to $CO_2$ synthon of from about 3:1 to about 10:1. For the preparation of carboxylated compounds having an unsymmetrical configuration, it is preferred to use a $CO_2$ synthon starting material other than an alkylene carbonate, e.g., dimethyl carbonate, or an equimolar or molar excess of any $CO_2$ synthon starting material with any active hydrogen-containing compound starting material, e.g., a molar ratio of active hydrogen-containing compound to $CO_2$ synthon of from about 0.1:1 to about 1:1.

It is appreciated that two or more $CO_2$ synthons can be reacted under conditions effective to produce a carboxylated compound.

The processes of this invention can be conducted in the presence of an inert diluent which can be either a liquid or gas. When a liquid diluent is employed, it should preferably be a good solvent for the starting materials, inert under the reaction conditions, and of such a nature that separation from the carboxylated product will not be difficult. For instance, the boiling points of the diluent and the carboxylated product should differ by an adequate amount and there should be no tendency of the diluent to form an azeotrope with the desired carboxylated product.

Examples of useful liquid diluents that meet the foregoing qualifications include benzene, toluene, xylene, ethylbenzene, anisole, heptane, octane, nonane, decane, dibutyl ether, and the like. Hydrocarbons are preferred.

Illustrative gaseous diluents include for example, nitrogen, methane, hydrogen, carbon monoxide or carbon dioxide. The gaseous diluent should of course be chosen so that it does not prevent the preparation of the desired carboxylated products.

While the use of such diluents may be beneficial, the processes of this invention can be operated using pure starting material(s) as a liquid or gaseous feed. The degree of dilution of the starting materials with various diluents may vary considerably depending upon any process constraints restricting the use of the diluent. For example, in commercial production, the use of very large quantities of some gaseous diluents may be disadvantageous due to the cost of pumping large volumes of the gaseous diluent and increased difficulty in isolating the carboxylated product, which increase the energy costs of the process. With liquid diluents, the use of very large quantities may be disadvantageous due to the energy cost associated with large recovery and recycle. If the processes of this invention are to be carried out using a gaseous diluent, in general it is recommended that the starting material(s) constitute from about 1 to about 95, and preferably about 5 to about 50, mole percent of the starting material/carrier feed. Increasing the dilution of the starting

material with a gaseous diluent such as hydrogen may tend to increase the selectivity of the reaction to the particular products desired. The amount of liquid diluent can vary widely, for instance, from no diluent to about 90 weight percent or greater of the total weight of the starting materials.

The carboxylated compounds produced by the processes of this invention can be separated by distillation. For example, a crude reaction product can be subjected to a distillation-separation at atmospheric or reduced pressure through a packed distillation column. Reactive distillation may be useful in conducting the reaction.

The processes of this invention may be carried out using, for example, a fixed bed reactor, a fluid bed reactor, or a slurry reactor. The optimum size and shape of the catalyst will depend on the type of reactor used. In general, for fluid bed reactors, a small, spherical catalyst particle is preferred for easy fluidization. With fixed bed reactors, larger catalyst particles are preferred so the back pressure within the reactor is kept reasonably low.

The processes of this invention can be conducted in a batch or continuous fashion, with recycle of unconsumed starting materials if required. The reaction can be conducted in a single reaction zone or in a plurality of reaction zones, in series or in parallel or it may be conducted batchwise or continuously in an elongated tubular zone or series of such zones. The materials of construction employed should be inert to the starting materials during the reaction and the fabrication of the equipment should be able to withstand the reaction temperatures and pressures. Means to introduce and/or adjust the quantity of starting materials or ingredients introduced batchwise or continuously into the reaction zone during the course of the reaction can be conveniently utilized in the processes especially to maintain the desired molar ratio of the starting materials. The reaction steps may be effected by the incremental addition of one of the starting materials to the other. Also, the reaction steps can be combined by the joint addition of the starting materials to the catalyst. When complete conversion is not desired or not obtainable, the starting materials can be separated from the carboxylated compound product, for example, by distillation, and the starting materials then recycled back into the reaction zone.

The processes of this invention are conducted for a period of time sufficient to produce the carboxylated compounds. The exact reaction time employed is dependent, in part, upon factors such as temperature, nature and proportion of starting materials, and the like. The reaction time will normally be within the range of from about one-half to about 100 hours or more, and preferably from less than about one to about ten hours.

The processes may be conducted in either glass lined, stainless steel or similar type reaction equipment. The reaction zone may be fitted with one or more internal and/or external heat exchanger(s) in order to control undue temperature fluctuations, or to prevent any possible "runaway" reaction temperatures.

Illustrative of suitable reactants in effecting the processes of this invention include by way of example:

UR - urea
DMC - dimethyl carbonate
PC - propylene carbonate
EC - ethylene carbonate
BEA - 2-butoxyethyl acetate
MEA - monoethanolamine
ET - ethanol
ME - methanol
MEL - 2-methoxyethanol
MEEL - 2-(2-methoxyethoxy)ethanol
AA - allyl alcohol

Illustrative of suitable products prepared by the processes of this invention include by way of example:

DMC - dimethyl carbonate
DEC - diethyl carbonate
BCMC - 2-butoxyethyl methyl carbonate
BBCC - bis(2-butoxyethyl)carbonate
OX - 2-oxazolidinone
DAC - diallyl carbonate
BMC - bis(2-methoxyethyl)carbonate
BMEEC - bis[2-(2-methoxyethoxy)ethyl)carbonate

Illustrative of permissible reactions encompassed within the scope of this invention include, for example, the following reactant/product combinations:

| REACTANT(S) | PRODUCT(S) |
|---|---|
| EC, ME | DMC |
| EC, ET | DEC |
| BEA, DMC | BCMC |
| BEA, DMC | BBCC |
| MEA, EC | OX |
| PC, ME | DMC |
| ME, UR | DMC |
| EC, MEL | BMC |
| EC, MEEL | BMEEC |
| AA, EC | DAC |

As used herein, the phrase "residue of an organic compound" is contemplated to include all permissible residues of organic compounds. In a broad aspect, the permissible residues include acyclic and cyclic, branched and unbranched, carbocyclic and heterocyclic, aromatic and nonaromatic residues of organic compounds. Illustrative organic compound residues include, for example, alkyl, aryl, cycloalkyl, heterocycloalkyl, alkyl(oxyalkylene), aryl(oxyalkylene), cycloalkyl(oxyalkylene), heterocycloalkyl-(oxyalkylene), hydroxy(alkyleneoxy) and the like. The permissible residues can be substituted or unsubstituted and the same or different for appropriate organic compounds. This invention is not intended to be limited in any manner by the permissible residues of organic compounds.

As used herein, the term "substituted" is contemplated to include all permissible substituents of organic compounds. In a broad aspect, the permissible substituents include acyclic and cyclic, branched and unbranched, carbocyclic and heterocyclic, aromatic and nonaromatic substituents of organic compounds. Illustrative substituents include, for example, alkyl, alkyloxy, aryl, aryloxy, hydroxy, hydroxyalkyl, amino, aminoalkyl, halogen and the like in which the number of carbons can range from 1 to about 20 or more, preferably from 1 to about 12. The permissible substituents can be one or more and the same or different for appropriate organic compounds. This invention is not intended to be limited in any manner by the permissible substituents of organic compounds.

Certain of the following examples are provided to further illustrate the processes of this invention.

Examples 1-6

The following procedure was used for the preparation of carboxylated compounds in Examples 1-6. The reactions were conducted in a Parr mini-reactor equipped with a 300 milliliter autoclave, a heating mantle, and a variable speed motor-driven stirrer. The reaction temperature was monitored by a Doric Trendicator 400A and cooling was performed by introducing cold water through a cooling loop. A total of 32.0 grams (1 mol) of methanol, 22.0 grams (0.25 mol) of ethylene carbonate, and 0.5 grams of catalyst (if used) were placed in the autoclave and the system was evacuated and purged with nitrogen through a three-way valve. A total of 10 psig of nitrogen was charged to the autoclave and the reactants heated to the indicated temperature in Table I below. After maintaining the reaction temperature for a period of 4 hours, the autoclave was cooled to room temperature and the product mixture analyzed by capillary gas chromatography (FID) using a DB-1701 column. The results are set forth in Table I.

The catalysts employed in Examples 1-6 were prepared as follows:

Catalyst A: Magnesium:aluminum mixed metal oxide (3:1 Mg(Al) obtained from United Catalysts Inc., Louisville, Kentucky.

Catalyst B: Sulfate modified calcium oxide.

Catalyst C: Zirconium dioxide pellets (150cc) were added to a saturated solution of ammonium sulphate in water. Enough solution was added to completely immerse the pellets. The slurry was allowed to stand at a temperature of 55 - 60 °C for a period of 8 hours. The catalyst was filtered, washed with water until the wash water was neutral, dried at a temperature of 100 °C, and then calcined at a temperature of 600 °C for a period of 6-8 hours.

Table I

| Example No. | 1 | 2 | 3 | 4 | 5 | 6 |
|---|---|---|---|---|---|---|
| **Process Parameters** | | | | | | |
| Temperature, °C | 100 | 150 | 100 | 150 | 150 | 150 |
| Duration of Run, hours | 4 | 4 | 4 | 4 | 4 | 4 |
| Catalyst Type | None | None | A | A | B | C |
| **Product Composition, area %** | | | | | | |
| Methanol | 79.56 | 65.05 | 75.37 | 59.19 | 54.24 | 57.65 |
| Ethylene carbonate | 16.86 | 14.60 | 12.53 | 8.66 | 14.76 | 11.29 |
| Dimethyl carbonate | 0.87 | 9.43 | 5.64 | 16.63 | 12.32 | 7.39 |
| Ethylene glycol | 0.70 | 9.35 | 4.12 | 13.99 | 17.15 | 14.45 |
| 2-Methoxyethanol | - | 0.15 | - | 0.16 | 0.12 | 3.57 |
| Others | 1.84 | 0.97 | 1.75 | 1.11 | 1.05 | 1.98 |
| Dimethyl carbonate/ Ethylene carbonate ratio | 0.05 | 0.65 | 0.45 | 1.92 | 0.83 | 0.65 |
| Ethylene glycol/ Ethylene carbonate ratio | 0.04 | 0.64 | 0.33 | 1.62 | 1.16 | 1.28 |

Examples 7-18

The following apparatus was used for the preparation of carboxylated compounds in Examples 7-18. An Applied Test Systems, Inc. Model 3620 Split Test Oven equipped with a preheater (stainless steel 1/8 inch o.d. x 2 feet) and 1/2 inch (o.d) stainless steel reactor tube (8 inch length) was packed with catalyst and heated to the desired reaction temperature using a Honeywell Dial-A-Trol temperature controller. The temperatures at the top of the reactor and the bottom were monitored using a digital temperature readout. The liquid feed was added (downflow) to the reactor via a Fluid Metering Inc. RP-G20 drive pump equipped with an 1/8 inch pump head. The system was maintained under nitrogen, which was introduced prior to the liquid preheater and was monitored with a rotometer. The product mixture was collected in a 100 milliliter round bottom flask, vented first to a dry ice/acetone trap and then a Firestone valve. Analysis was performed by capillary gas chromatography (FID) using a DB-1701 column.

For Examples 7-14, a solution containing methanol and ethylene carbonate in the amount indicated in

Table II below was passed through a catalyst bed containing 5.0 grams of a magnesium:aluminum mixed metal oxide (3/16 inch tablets, Mg/Al 3:1) at the reaction temperature indicated in Table II and at a liquid feed rate of about 0.15-0.2 milliliters per minute. The feed was continued for a period of 2 hours. The results are given in Table II.

For Examples 15-18, a solution containing ethanol and ethylene carbonate in the amount indicated in Table III below was passed through a catalyst bed containing 5.0 grams of a magnesium:aluminum mixed metal oxide (3/16 inch tablets, Mg/Al 3:1) at the reaction temperature indicated in Table III and at a liquid feed rate of about 0.15-0.2 milliliters per minute. The feed was continued for a period of 2 hours. The results are given in Table III.

Table II

| Example No. | 7 | 8 | 9 | 10 | 11 | 12 | 13 | 14 |
|---|---|---|---|---|---|---|---|---|
| Process Parameters | | | | | | | | |
| Temperature, °C | 150 | 175 | 125 | 150 | 175 | 150 | 175 | 200 |
| Methanol/Ethylene carbonate, mole ratio | 6.06 | 6.06 | 12.15 | 12.15 | 12.15 | 18 | 18 | 18 |
| Product Composition, area % | | | | | | | | |
| Methanol | 85.90 | 86.58 | 94.04 | 92.21 | 92.20 | 95.06 | 93.82 | 92.81 |
| Ethylene carbonate | 5.68 | 2.43 | 1.85 | 2.47 | 0.60 | 1.12 | 0.73 | 1.32 |
| Dimethyl carbonate | 3.52 | 3.89 | 2.50 | 2.65 | 3.18 | 2.00 | 2.44 | 0.91 |
| Ethylene glycol | 2.28 | 1.03 | 0.76 | 1.42 | 0.74 | 0.81 | 0.65 | 0.92 |
| 2-Methoxyethanol | 0.40 | 1.03 | 0.06 | 0.23 | 1.01 | 0.17 | 0.70 | 1.04 |
| Others | 1.57 | 3.40 | 0.46 | 0.80 | 1.05 | 0.52 | 1.43 | 2.32 |
| Dimethyl carbonate/ Ethylene carbonate ratio | 0.62 | 1.60 | 1.35 | 1.07 | 5.30 | 1.79 | 3.34 | 0.69 |
| Ethylene glycol/ Ethylene carbonate ratio | 0.40 | 0.42 | 0.41 | 0.57 | 1.23 | 0.72 | 0.89 | 0.70 |

**Table III**

| Example No. | 15 | 16 | 17 | 18 |
|---|---|---|---|---|
| **Process Parameters** | | | | |
| Temperature, °C | 150 | 175 | 125 | 150 |
| Ethanol/Ethylene carbonate, mole ratio | 6 | 6 | 12 | 12 |
| **Product Composition, area %** | | | | |
| Ethanol | 92.20 | 89.61 | 95.52 | 94.76 |
| Ethylene carbonate | 3.62 | 3.17 | 1.72 | 1.82 |
| Diethyl carbonate | 1.73 | 2.68 | 1.10 | 1.62 |
| Ethylene glycol | 0.64 | 0.47 | 0.35 | 0.70 |
| 2-Ethoxyethanol | 0.13 | 0.42 | 0.03 | 0.12 |
| Others | 0.90 | 1.53 | 0.57 | 0.64 |
| Diethyl carbonate/ Ethylene carbonate ratio | 0.48 | 0.85 | 0.64 | 0.89 |
| Ethylene glycol/ Ethylene carbonate ratio | 0.18 | 0.15 | 0.20 | 0.38 |

Example 19

Using the apparatus employed in Examples 7-18, a solution containing 64.0 grams (0.4 mol) of 2-butoxyethanol acetate and 18.02 grams (0.2 mol) of dimethyl carbonate was passed through a catalyst bed containing 11.5 grams of a magnesium:aluminum mixed metal oxide (3/16 inch tablets, Mg/Al 3:1) at a temperature of 150 °C and a flow rate of about 0.3 milliliters per minute. After a period of 1 hour, a total of 15.6 grams of product was obtained. The results are given in Table IV below.

Table IV

| Product Composition, area % | |
|---|---|
| 2-Butoxyethanol acetate | 59.83 |
| Dimethyl carbonate | 4.87 |
| Bis(2-butoxyethyl)carbonate | 13.71 |
| 2-Butoxyethyl methyl carbonate | 9.92 |
| 2-Butoxyethanol | 2.23 |
| Methyl acetate | 8.38 |

Example 20

Using the apparatus employed in Examples 7-18, a solution containing 32.0 grams (0.2 mol) of 2-butoxyethanol acetate and 18.02 grams (0.2 mol) of dimethyl carbonate was passed throught a catalyst bed containing 10.0 grams of a magnesium:aluminum mixed metal oxide (3/16 inch tablets, Mg/Al 3:1) at a temperature of 150°C and a flow rate of about 0.3 milliliters per minute. After a period of 1 hour, a total of 12.4 grams of product was obtained. The results are given in Table V below.

Table V

| Product Composition, area % | |
|---|---|
| 2-Butoxyethanol acetate | 47.18 |
| Dimethyl carbonate | 10.82 |
| Bis(2-butoxyethyl)carbonate | 14.00 |
| 2-Butoxyethyl methyl carbonate | 12.68 |
| 2-Butoxyethanol | 2.19 |
| Methyl acetate | 11.90 |

Although the invention has been illustrated by certain of the preceding examples, it is not to be construed as being limited thereby; but rather, the invention encompasses the generic area as hereinbefore disclosed. Various modifications and embodiments can be made without departing from the spirit and scope thereof.

**Claims**

**1.** A process for preparing carboxylated compounds which comprises contacting an active hydrogen-containing compound with $CO_2$ synthon in the presence of a mixed metal oxide catalyst or a modified bimetallic or polymetallic catalyst under conditions effective to produce the carboxylated compound.

**2.** The process of claim 1 wherein the active hydrogen-containing compound comprises a substituted or unsubstituted alcohol, phenol, carboxylic acid, amine, thiophenol, mercaptan or amide.

**3.** The process of claim 1 wherein the active hydrogen-containing compound comprises a substituted or unsubstituted active hydrogen-containing compound.

**4.** The process of claim 1 wherein the active hydrogen-containing compound comprises methanol, ethanol, 2-(2-methoxyethoxy)ethanol, 2-(2-butoxyethoxy)ethanol, 2-methoxyethanol, a poly(oxyalkylene)-glycol, an alkoxy or allyloxy poly(oxyalkylene)glycol, an alkoxy or allyloxy poly(oxyethylene)-(oxypropylene)glycol, ethylene glycol, diethylene glycol, triethylene glycol, tetraethylene glycol, pentaethylene glycol, hexaethylene glycol, a polyethylene glycol, diethanolamine, triethanolamine, monoethanolamine or aminoethylethanolamine.

**5.** The process of claim 1 wherein the $CO_2$ synthon comprises a substituted or unsubstituted carbonate, chlorocarbonate, carbonic acid, carbamate, carbamic acid, oxalate, 2-oxazolidinone, urea, ester, phosgene, chloroformate, carbon dioxide, orthocarboxylate, sulfurous acid or sulfurous acid ester.

**6.** The process of claim 1 wherein the $CO_2$ synthon comprises dimethyl carbonate, diethyl carbonate, ethylene carbonate or propylene carbonate.

**7.** The process of claim 1 wherein the mixed metal oxide catalyst comprises two or more Group IA metal oxides, Group IIA metal oxides, Group IIIB metal oxides, Group IVB metal oxides, Group VB metal oxides, Group VIB metal oxides, Group VIIB metal oxides, Group VIII metal oxides, Group IB metal oxides, Group IIB metal oxides, Group IIIA metal oxides, Group IVA metal oxides, Group VA metal oxides or Group VIA metal oxides.

**8.** The process of claim 7 wherein the mixed metal oxide catalyst comprises two or more oxides of magnesium, aluminum, calcium, strontium, gallium, beryllium, barium, scandium, yttrium, lanthanum, cerium, gadolinium, terbium, dysprosium, holmium, erbium, thulium, lutetium, ytterbium, niobium, tantalum, chromium, molybdenum, tungsten, titanium, zirconium, hafnium, vanadium, iron, cobalt, nickel, zinc, silver, cadmium, boron, indium, silicon, germanium, tin, lead, arsenic, antimony and bismuth.

**9.** The process of claim 1 wherein the mixed metal oxide catalyst comprises at least one Group IIA metal oxide.

**10.** The process of claim 1 wherein the mixed metal oxide catalyst comprises a Group IIA metal oxide and a Group IIIA metal oxide.

**11.** The process of claim 1 wherein the mixed metal oxide catalyst comprises a Group IIA metal oxide and a Group IIIB metal oxide.

**12.** The process of claim 1 wherein the mixed metal oxide catalyst comprises magnesium oxide and aluminum oxide.

**13.** The process of claim 1 wherein the mixed metal oxide catalyst comprises a high surface area mixed metal oxide.

**14.** The process of claim 1 wherein the mixed metal oxide catalyst has a surface area greater than about 50 $m^2$/gm.

**15.** The process of claim 9 wherein the Group IIA metal oxide comprises from about 10 weight percent to about 90 weight percent of the weight of the catalyst.

**16.** The process of claim 1 wherein the mixed metal oxide catalyst is associated with a support material.

**17.** The process of claim 13 wherein the support comprises an alumina material or an alumina-silica material.

**18.** The process of claim 13 wherein the support comprises an silica material or a silica-alumina material.

**19.** The process of claim 13 wherein the support comprises from about 2 to about 50 percent by weight of the mixed metal oxide catalyst.

**20.** The process of claim 1 wherein the mixed metal oxide catalyst comprises:
(a) a material having the formula

$$M_x^{2+} Q_y^{3+} (OH)_{2x+3y-nz} A_z^{n-} \cdot a\ H_2O \qquad (I)$$

wherein M is at least one divalent metal cation; Q is at least one trivalent metal cation; and A is at

least one anion providing a valence ($n^-$), wherein n is 1 to 4 and wherein a is a positive number, M, Q and A are provided in a proportion such that x/y is a number equal to or greater than 1, z has a value greater than zero and 2x + 3y-nz is a positive number, or

(b) a material prepared by calcining the material of formula (I) having the formula

$$M_x^{2+} Q_y^{3+} (O)_{(2x+3y-nz)/2} D_z^{n-} \qquad (II)$$

wherein M, Q, x, y, z and n have the same meanings defined above in connection with formula (I), and D is at least one nonvolatile anion.

**21.** The process of claim 20 wherein x/y is a number between 1 and 12 and z has a value which satisfies the relationship: x/z is between n and 12n.

**22.** The process of claim 20 wherein A is selected from the group consisting of carbonate, halide, phosphite, phosphate, chromate, sulfate, hydroxide, oxalate, acetate, nitrate, hexanoate, sebacate, vanadate, molybdate, tungstate and ferrocyanate.

**23.** The process of claim 20 wherein D is selected from the group consisting of halides, phosphite, phosphate, vanadate, molybdate, tungstate, sulfite, sulfate, chromate, arsenate, borate and chlorate.

**24.** The process of claim 20 wherein x/y is a number between 1 and 6 and z has a value which satisfies the relationship: x/z is between n and 6n.

**25.** The process of claim 20 wherein said material prepared by calcining the material of formula (I) has been heat treated at a temperature in the range of 200 °C to 800 °C for 12 to 24 hours.

**26.** The process of claim 20 wherein M is magnesium and Q is aluminum.

**27.** The process of claim 1 wherein the modified bimetallic or polymetallic catalyst comprises a material having the formula

$$[R_4\text{-}X_1\text{-}M_1]_f\text{-}Y_1\text{-}[M_3\text{-}Y_2]_j\text{-}[M_2\text{-}X_2\text{-}R_5]_g$$

wherein:

$R_4$ and $R_5$ are independently hydrogen or the residue of an organic compound having at least one active hydrogen;

$X_1$ and $X_2$ are independently oxygen, sulfur or nitrogen;

$M_1$, $M_2$ and $M_3$ are independently a divalent or polyvalent metal;

$Y_1$ and $Y_2$ are independently a divalent or polyvalent oxyacid anion of valence 2 to 6, oxygen, sulfur or nitrogen provided at least one of $Y_1$ and $Y_2$ is a divalent or polyvalent oxyacid anion of valence 2 to 6;

j is an integer having a value of from 0 to about 100; and

f and g are integers having a value such that the sum f + g is equal to the valence of $Y_1$ when j is a value of 0, and f and g are integers having a value such that the sum f + g is equal to the valence of $Y_1$ plus $[M_3\text{-}Y_2]_j$ when j is a value other than 0.

**28.** The process of claim 27 wherein the divalent or polyvalent oxyacid anion comprises sulfates, phosphates, manganates, titanates, tantalates, molybdates, vanadates, chromates, zirconates and polyphosphates.

**29.** The process of claim 27 wherein the divalent or polyvalent metal comprises calcium, magnesium, strontium, barium, lanthanum, titanium, zirconium, hafnium, vanadium, niobium, tantalum, chromium, molybdenum, tungsten, manganese, iron, cobalt, nickel, copper, zinc, cadmium, mercury, boron, aluminum, gallium, indium, thallium, carbon, silicon, germanium, tin, lead, phosphorus, arsenic, antimony, sulfur, selenium and tellurium.

**30.** The process of claim 27 wherein the modified bimetallic or polymetallic catalyst comprises a modified calcium catalyst or a modified magnesium catalyst.

**31.** The process of claim 1 wherein the carboxylated compound comprises a substituted or unsubstituted carboxylated compound.

**32.** The process of claim 1 wherein the carboxylated compound comprises a material having the formulae:

$ROC(O)R_1$ or $ROC(O)R_2$

wherein R is the residue of an organic compound, $R_1$ is the residue of an organic compound, $R_2$ is the residue of an organic compound, and optionally the R and $R_1$ substituents or the R and $R_2$ substituents are bridged together to complete a heterocycloalkyl ring which can be substituted or unsubstituted.

**33.** The process of claim 1 wherein the carboxylated compound comprises a material having the formulae:

$ROC(O)OR_1$ or $ROC(O)OR_2$

wherein R is the residue of an organic compound, $R_1$ is the residue of an organic compound, $R_2$ is the residue of an organic compound, and optionally the R and $R_1$ substituents or the R and $R_2$ substituents are bridged together to complete a heterocycloalkyl ring which can be substituted or unsubstituted.

**34.** The process of claim 1 wherein the carboxylated compound comprises a material having the formulae:

$RN(C(O)OR_1)R$ or $RN(C(O)OR_2)R$

wherein R is the residue of an organic compound, $R_1$ is the residue of an organic compound, $R_2$ is the residue of an organic compound, and optionally the R substituents or the R and $R_1$ substituents or the R and $R_2$ substituents are bridged together to complete a heterocycloalkyl ring which can be substituted or unsubstituted.

**35.** The process of claim 25 wherein the carboxylated compound comprises dimethyl carbonate, diethyl carbonate, 2-oxazolidinone, diallyl carbonate, bis(2-methoxyethyl)carbonate, bis[2-(2-methoxyethoxy)-ethyl]carbonate, 2-butoxyethyl methyl carbonate or bis(2-butoxyethyl)carbonate.

**36.** A process for preparing carboxylated compounds which comprises contacting one or more $CO_2$ synthons in the presence of a mixed metal oxide catalyst or a modified bimetallic or polymetallic catalyst under conditions effective to produce the carboxylated compound.

**37.** The process of claim 36 wherein the $CO_2$ synthon comprises a substituted or unsubstituted carbonate, chlorocarbonate, carbonic acid, carbamate, carbamic acid, oxalate, 2-oxazolidinone, urea, ester, phosgene, choroformate, carbon dioxide, orthocarboxylate, sulfurous acid or sulfurous acid ester.

**38.** The process of claim 36 wherein the $CO_2$ synthon comprises dimethyl carbonate, diethyl carbonate, ethylene carbonate or propylene carbonate.

**39.** The process of claim 36 wherein the mixed metal oxide catalyst comprises two or more Group IA metal oxides, Group IIA metal oxides, Group IIIB metal oxides, Group IVB metal oxides, Group VB metal oxides, Group VIB metal oxides, Group VIIB metal oxides, Group VIII metal oxides, Group IB metal oxides, Group IIB metal oxides, Group IIIA metal oxides, Group IVA metal oxides, Group VA metal oxides or Group VIA metal oxides.

**40.** The process of claim 39 wherein the mixed metal oxide catalyst comprises two or more oxides of magnesium, aluminum, calcium, strontium, gallium, beryllium, barium, scandium, yttrium, lanthanum, cerium, gadolinium, terbium, dysprosium, holmium, erbium, thulium, lutetium, ytterbium, niobium, tantalum, chromium, molybdenum, tungsten, titanium, zirconium, hafnium, vanadium, iron, cobalt, nickel, zinc, silver, cadmium, boron, indium, silicon, germanium, tin, lead, arsenic, antimony and bismuth.

**41.** The process of claim 36 wherein the mixed metal oxide catalyst comprises at least one Group IIA metal oxide.

**42.** The process of claim 36 wherein the mixed metal oxide catalyst comprises a Group IIA metal oxide and a Group IIIA metal oxide.

**43.** The process of claim 36 wherein the mixed metal oxide catalyst comprises a Group IIA metal oxide and a Group IIIB metal oxide.

**44.** The process of claim 36 wherein the mixed metal oxide catalyst comprises magnesium oxide and aluminum oxide.

**45.** The process of claim 36 wherein the modified bimetallic or polymetallic catalyst comprises a material having the formula

$$[R_4\text{-}X_1\text{-}M_1]_f\text{-}Y_1\text{-}[M_3\text{-}Y_2]_j\text{-}[M_2\text{-}X_2\text{-}R_5]_g$$

wherein:

$R_4$ and $R_5$ are independently hydrogen or the residue of an organic compound having at least one active hydrogen;

$X_1$ and $X_2$ are independently oxygen, sulfur or nitrogen;

$M_1$, $M_2$ and $M_3$ are independently a divalent or polyvalent metal;

$Y_1$ and $Y_2$ are independently a divalent or polyvalent oxyacid anion of valence 2 to 6, oxygen, sulfur or nitrogen provided at least one of $Y_1$ and $Y_2$ is a divalent or polyvalent oxyacid anion of valence 2 to 6;

j is an integer having a value of from 0 to about 100; and

f and g are integers having a value such that the sum f + g is equal to the valence of $Y_1$ when j is a value of 0, and f and g are integers having a value such that the sum f + g is equal to the valence of $Y_1$ plus $[M_3\text{-}Y_2]_j$ when j is a value other than 0.

**46.** The process of claim 45 wherein the divalent or polyvalent oxyacid anion comprises sulfates, phosphates, manganates, titanates, tantalates, molybdates, vanadates, chromates, zirconates and polyphosphates.

**47.** The process of claim 45 wherein the divalent or polyvalent metal comprises calcium, magnesium, strontium, barium, lanthanum, titanium, zirconium, hafnium, vanadium, niobium, tantalum, chromium, molybdenum, tungsten, manganese, iron, cobalt, nickel, copper, zinc, cadmium, mercury, boron, aluminum, gallium, indium, thallium, carbon, silicon, germanium, tin, lead, phosphorus, arsenic, antimony, sulfur, selenium and tellurium.

**48.** The process of claim 36 wherein the modified bimetallic or polymetallic catalyst comprises a modified calcium catalyst or a modified magnesium catalyst.

**49.** The process of claim 36 wherein the carboxylated compound comprises a substituted or unsubstituted carboxylated compound.

**50.** A process for preparing a dialkyl carbonate which comprises contacting an alkylene carbonate in the presence of a mixed metal oxide catalyst or a modified bimetallic or polymetallic catalyst under conditions effective to produce the dialkyl carbonate.

**51.** The process of claim 50 wherein the alkylene carbonate comprises ethylene carbonate or propylene carbonate.

**52.** The process of claim 50 wherein the mixed metal oxide catalyst comprises magnesium oxide and aluminum oxide.

**53.** The process of claim 50 wherein the modified bimetallic or polymetallic catalyst comprises a modified calcium catalyst or a modified magnesium catalyst.

**54.** The process of claim 50 wherein the dialkyl carbonate comprises dimethyl carbonate or diethyl carbonate.